# EUROPEAN PATENT APPLICATION

(11) **EP 1 275 368 A1**
(43) Date of publication of application: **15.01.2003**
(21) Application number: 01117174.1
(22) Date of filing: 14.07.2001
(51) Int. Cl.: A61K 7/46, A61L 9/12, C09D 11/02

(54) **Composition for adding perfumes to water soluble films**

(71) Applicant: Givaudan SA, 1214 Vernier (CH)
(72) Inventor: Quellet, Christian, 2502 Biel (CH); Balmer, Marc, 8103 Unterengstringen (CH)
(74) Representative: Simmons, John Murray, Dr.

(57) **Abstract**

The invention is concerned with a perfume composition comprising perfume, water and a surfactant and/or hydrocolloid. The composition may be in the form of a dispersion of perfume in water, or an oil-in-water emulsion, and is useful as a means of adding a perfume to a water-soluble polymeric film without adversely affecting the properties of said film.

## Description

This invention is concerned with a perfume composition and the application of the composition onto a water-soluble polymeric film.

Water-soluble polymeric films are being used for packaging applications. Such films are particularly useful for encapsulating solid actives, such as detergent powders, or nonaqueous liquid actives such as liquid detergents and conditioners and the like. The water-soluble films provide protection for, and easy handling of, the actives, as well as permitting fast delivery of the actives in aqueous media such as wash water and conditioning baths. Typical polymers finding use in these applications are selected from polymers and copolymers based on polyvinylaclohol or thermoplastic starch derivatives.

A problem with water-soluble polymeric films however, is their high permeability with respect to small hydrophilic molecules such as short-chain alcohols, amines or carboxylic acids which may be found in liquid actives for example, and which may impart unpleasant odours to the films. Furthermore, certain water-soluble film polymers, e.g. most polyvinylalcohol polymers and copolymers have a characteristic acetic acid smell due to the chemical degradation of non-hydrolysed monomeric vinylacetate units and the presence of residual sodium acetate in the polymer.

The addition of perfume to such films is desirable to counteract the malodour, and moreover to present an aesthetic olfactory signal to the user upon opening a package containing an encapsulated liquid detergent for example.

Perfume may be added to a film either by direct addition of the perfume to a bulk water-soluble film-forming polymer, or by applying perfume to pre-formed sheets or finished articles comprising the water-soluble polymeric films. Addition of neat perfume to the water-soluble bulk polymer however, is not a satisfactory solution because any residual off-cuts of bulk polymer may not be easily recycled without altering the olfactory quality of the recycled polymer. On the other hand, application of perfume, e.g. by spraying or printing onto pre-formed polymer sheets or finished articles may be undesirable for many reasons: Spraying flammable perfume aerosols has obvious safety implications which would add to the complexity and cost of manufacture, whereas printing perfumes, that may contain significant amounts of hydrophobic perfume ingredients, directly onto polymer sheets or finished articles may leave a surface layer of perfume that will affect not only the visual and tactile aspects of the polymer, but also permit of rapid evaporation of the volatile components of the perfume layer with concomitant rapid reduction in the olfactive impact during storage.

A composition has now been found that can be applied to water-soluble polymeric films and which presents essentially none of the disadvantages referred to above. The invention therefore provides in one of its aspects a perfume composition for adding perfume to a water-soluble polymeric film comprising a perfume, water and a polymer surfactant and/or a hydrocolloid.

The composition according to the invention is possessed of many advantages: It may be applied directly to a water-soluble film without altering the physical and chemical properties of the film, for example the dissolution rate of the film in aqueous media is not adversely affected and the visual and tactile appearance of the film remains unchanged. Furthermore, the evaporation rate of the perfume is reduced relative to perfumes added neat to said films, which in turn provides for a longer, more controlled release of the perfume. By "neat" is meant a perfume consisting only of perfume ingredients or perfume ingredients dissolved or dispersed in a base, e.g. an alcoholic base.

In a composition according to the present invention the perfume is dispersed in the water. The perfume may be in the form of fine solid particles or liquid droplets. When the perfume is in the form of solid particles the composition is considered to be a dispersion of perfume in water, whereas when the perfume is in the form of liquid droplets, the composition is an oil-in-water emulsion.

It is a feature of the present invention that the composition may be applied to a water-soluble polymer film and release the perfume in a controlled manner over a prolonged period of time, thereby ensuring that the film retains a pleasant smell even after prolonged periods of storage, e.g. up to 3 months. Control of the release of perfume may be achieved by altering the particle size of the perfume particles or droplets. Typically, the particle or droplet sizes employed range from 1 to 5 microns, more particularly 1 to 2 microns. We have found that the larger the particles or droplets the slower is the release, whereas smaller particle or droplet size favours relatively rapid release, and the skilled person is capable of experimenting without undue burden to find the appropriate particle or droplet size to achieve a desired release rate for a given application or end use of the perfume composition. A composition according to the present invention may be applied, e.g. sprayed, printed or coated onto sheets of water-soluble polymer film either before the film is cut or shaped into finished articles, or it may be applied to the film in a finished article. Standard aerosol chambers, stampers or rollers may be employed to effect application of the composition. By virtue of the composition being water-based, flammability hazards associated with aerosol application of perfume may be reduced or obviated compared with the hazards associated with the application of neat perfumes. It is also thought that the surfactant and/or hydrocolloid by virtue of surrounding the perfume particles or droplets acts to reduce the vapour pressure of the perfume and thereby also contribute to the reduction in flammability hazard.

In a particular embodiment of the present invention, the composition may be added to a water-based ink to give a perfumed ink composition that can be printed or otherwise applied to a water-soluble polymer film using conventional application techniques, such as spraying from ink jet nozzles, or stampers and applicator rolls to produce perfumed writing, logos, pictures and the like on the water-soluble polymer films.

The composition of the present invention may be applied to any natural or synthetic water-soluble polymers known for use in the manufacture of water-soluble films. In particular one can mention polymers such as polyvinylalcohols (PVA), modified PVA, polyvinylpyrollidone, starch and starch derivatives, and modified cellulose.

Perfumes for use in the composition should be sparingly soluble in water, that is, their solubility should be such that it is possible to form stable dispersions or oil-in-water emulsions. In particular, perfumes are composed of ingredients having a calculated Log P (c. log P) value of about 2.5 or greater, and more particularly at least more than 50%, e.g. 75% or more of the perfume by weight is composed of perfume ingredients having a c log P of 2.5 or greater. The term « c. log P » is a parameter known to the skilled person and is a measure of the hydrophobicity of an agent and represents the calculated partition coefficient of an agent in octanol/water.

The amount of perfume present in a composition according to the invention may vary from about 5 to about 40% by weight, in particular 15 to 30%, more particularly 2 to 10%, by weight. The perfume may consist of a single perfume ingredient or a mixture of perfume ingredients.

The surfactant may be any of those surfactants known in the art for stabilising dispersions of solid particles in water or stabilising in oil-in-water emulsions. Particular surfactants are polymer surfactants selected from polyoxyethylene polymers, poly(ethyleneoxide)-b-propylene oxide) block copolymers, or silicone-based surface active polymers. One problem with water-soluble polymer films is their tendency of « blocking ». Blocking is a term used to describe the process of adjacent films sticking to each other, for example during storage. This is a particular problem at high relative humidity conditions, for example greater than 70% relatively humidity. Accordingly, it is often the case that measures against blocking may be required, for example interposing between adjacent film surfaces, non-stick materials, e.g. non-stick paper sheets and the like. However, this adds complexity to the manufacture and storage of such films or finished articles made therefrom and it would be desirable to avoid this precaution. Surprisingly, it has now been found that the use of the aforementioned silicone-based surfactants may reduce significantly blocking of the films. For this reason, it is preferred to use a silicone-based surfactant in a composition according to the invention.

Preferred silicone-based surfactants may be selected from modified polydimethylsiloxanes (PDMS), and in particular PDMS copolymers with polyether units, those units either being present in the copolymers as side-chains or as end-caps or both. Other useful PDMS may be modified with grafted sugar and/or polysaccharide moieties. More preferred PDMS are water-soluble poly(ethyleneoxide-graft-dimethylsiloxane) copolymers. Most preferred are the polymers available commercially as Tegopren 5851 and Tegopren 5884.

Typically the surfactant may be present in a composition according to the invention in amounts of from about 3 to about 6% by weight of the total weight of the composition.

As hydrocolloids, there may be mentioned any of those hydrocolloids useful in stabilising dispersions or oil-in-water emulsions. In particular one can mention xanthan gums, propylene glycol alginate, guar gum, gum tragarcanth, locust bean gum, gellan gum, gum arabic, cellulosic gums, cellulose derivatives such as hydroxypropylmethylcellulose, carboxymethyl cellulose and microcrystalline cellulose, modified food starches or polyvinyl alcohols. Preferred hydrocolloids, however, may be selected from polyvinylalcohols, in particular those having a viscosity number of not greater than 20 and more particularly not higher than 8. The hydrocolloid may have a degree of hydrolysis of between 80 and 99%, more particularly between 82 and 92%. A particularly preferred hydrocolloid is the polyvinylalcohol available commercially as MOWIOL 4/8.

The hydrocolloid may be present in the composition in an amount of about 1 to 6%, more particularly 3 to 6% by weight based on the total weight of the composition.

In a particular embodiment, the surfactant and hydrocolloid may be used together as an emulsifier mix. In such an embodiment it is preferred to use at least one of the PDMS and at least one polyvinylalcohol hydrocolloid aforementioned in admixture. An emulsifier mix may be present in amounts of about 3 to 6% by weight based on the total weight of the composition. Preferably, the emulsifier mix contains a surfactant in an amount of 50 up to about 100%, more particularly about 80 up to about 100%, e.g. 50 to 99.9%; the remaining mass of the emulsifier mix being provided by the hydrocolloid.

A composition according to the invention may comprise other adjuvants and additives, for example a co-solubiliser selected from water-soluble organic solvents such as glycol ethers and alcohols; and additional surface-active species such as surfactants and emulsifiers with a hydrophobic to hydrophilic balance of about 10 to 18, more particularly ethoxylated fatty acids and polyether modified silicones. A particularly preferred additional surface-active species may be selected from an ethoxylated castor oil containing 40 ethylene oxide units, available commercially under as CHREMOPHORE RH 40.

Compositions according to the invention may be formed by any of the classical methods known in the art for forming dispersions in aqueous media or forming oil-in-water emulsions.

There now follows a series of examples that serve to illustrate the invention.

### Example 1

### Preparation of a composition

5 parts of emulsifier consisting of surfactant and/or hydrocolloid (for proportions see table) is added under stirring to 70 parts of deionized water and stirred until a clear aqueous solution is obtained. 25 parts of perfume are then added to the aqueous solution and homogenised for 5 minutes with a high shear mixer (Ultra Turrax) to form an oil-in-water emulsion.

| Emulsion | Perfume (%) | Polymer surfactant (%) | Hydrocolloid (%) | Water (%) |
|---|---|---|---|---|
| 1 | 25 | 5 (Tegoprene® 5884) | - | 70 |
| 2 | 25 | 2,5 (Tegoprene® 5851) | 2.5 (Mowiol® 4-88) | 70 |
| 3 | 25 | - | 14 (Mowiol® 4-88) | 61 |
| 4 | 25 | - | 10 (Mowiol® 4-88) | 65 |

### Example 2

### Assessment of film "blocking"

A water-soluble polyvinylalcohol film was divided into six rectangular pieces of equal size (10 x 5 cm). 8 mg to 13 mg an emulsion (Emulsions 1 through 4 of Example 1) was sprayed on six pieces of polyvinylalcohol film and the initial weight increase taken as an accurate measure of the deposited material. After drying for approximately 1.5 min, the films were folded out, sprayed face against sprayed face, and stored under a loaded glass plate (15g/cm²) for two days. The glass plate was then removed and the films separated from each other by hand. Film tear was taken as evidence of blocking.

| Emulsion (sprayed) | |
|---|---|
| Emulsion 1 8.1 mg | No visible defect |
| Emulsion 1 9.7 mg | No visible defect |
| Emulsion 2 13.0 mg | No visible defect |
| Emulsion 3 10 mg | Partially torn |
| Emulsion 4 10 mg | Partially torn |
| No Emulsion | torn |
| No Emulsion | torn |

The example shows that emulsions containing a water-soluble silicone-based emulsifier reduce or prevent film blocking.

### Example 3

### Dissolution measurement of treated polyvinylalcohol films

500 ml of water were poured into a 1 L beaker on a mechanical stirrer. The stirring speed was 170 rpm. Water-soluble polyvinylalcohol films strips (25 cm²) sprayed with emulsion 1 were placed into the beaker and immersed. The time till the film decomposed and completely dissolved was measured.

| Untreated Film | Decomposed (seconds) | Dissolved (seconds) |
|---|---|---|
| Strip 1 | 30 | 220 |
| Strip 2 | 22 | 170 |
| Strip 3 | 32 | 200 |

| Treated film (sprayed) c. 10mg Emulsion 1 | Decomposed (seconds) | Dissolved (seconds) |
|---|---|---|
| Strip 4 | 25 | 140 |
| Strip 5 | 24 | 74 |
| Strip 6 | 25 | 110 |
| Strip 7 | 28 | 175 |

This Example demonstrates that a sprayed-on emulsion does not delay the dispersion and dissolution of the films in water.

### Example 4

### Olfactory Assessment

Emulsion 1 was sprayed onto one side of a polyvinylalcohol film at a level of 7.0g (1.75g perfume) per square metre. The film was stored under air at room temperature and assessed regularly during storage.

| Time | Treated Film (Sprayed) c. 10mg Emulsion | Treated film (Sprayed) c. 10mg alcohol with the same perfume concentration |
|---|---|---|
| One day | Strong | Very strong |
| One week | Strong | Strong |
| One month | Strong | Weak |
| Three months | Strong | Not noticeable |

## Claims

1. A perfume composition for adding to a water-soluble polymeric film comprising a perfume, water and a surfactant and/or hydrocolloid.

2. A composition according to claim 1 in the form of an oil-in-water emulsion or a dispersion of perfume in water.

3. A composition according to claim 2 wherein the perfume is in the form of particles or droplets of 1 to 5 microns in size.

4. A composition according to any of the preceding claims wherein the surfactant is selected from a silicone-based surface active polymer, in particular a polydimethylsiloxane copolymer comprising polyether units, said units being present as side-chains or end-caps or both.

5. A composition according to any of the preceding claims wherein the hydrocolloid is a polyvinylalcohol, in particular having a viscosity number of not greater than 20, and a degree of hydrolysis of between 80 and 99%.

6. A composition according to any of the preceding claims comprising a surfactant as described in claim 4 and a hydrocolloid as described in claim 5 wherein together they are present in an amount of about 3 to 6% by weight of the composition.

7. The use of a composition as defined in any of the preceding claims in the application of a perfume to a water-soluble polymeric film, in particular a polyvinylalcohol water-soluble polymeric film.

8. A method of prolonging the release of a perfume from a water-soluble polymeric film or a finished article comprising said film for a period of at least 3 months, said method comprising the step of applying a composition as defined in any of the claims 1 to 6 to a water-soluble polymeric film or finished article.

9. A water-soluble polymeric film or a finished article comprising said film, comprising a perfume applied using a composition as defined in any of the claims 1 to 6.

10. A water-soluble polymeric film or a finished article comprising said film containing a perfume, which film or article when placed in an aqueous medium decomposes within 20 to 35 seconds and dissolves in 70 to 250 seconds.

11. A water-based ink composition comprising a perfume composition as described in any of the claims 1 to 6.
